# EUROPEAN PATENT APPLICATION

(11) **EP 1 388 352 A1**
(43) Date of publication of application: **11.02.2004**
(21) Application number: 02292830.3
(22) Date of filing: 14.11.2002
(51) Int. Cl.: A61P 3/04, A61K 45/06, A61K 31/216, A61K 31/4439

(54) **Use of a ppar-alpha agonist to treat patients suffering from weight gain associated with a ppar-gamma agonist treatment**

(30) Priority: 08.08.2002 EP 02291994
(71) Applicant: LABORATOIRES FOURNIER S.A., 21100 Dijon (FR)
(72) Inventor: Junien, jean-Louis, 92310 Sevres (FR); Edgar, Alan, 21490 Saint Julien (FR); Chaput, Evelyne, 21000 Dijon (FR)
(74) Representative: Nevant, Marc

(57) **Abstract**

The present invention relates to the use of a pparα agonist to treat patients suffering from weight gain associated with a pparγ agonist treatment.

## Description

The present invention relates to the use of a PPARα agonist to treat patients suffering from weight gain associated with a PPARγ agonist treatment.

Great advances have been made in the management of diabetes during the past decade. Whereas only one class of oral medications (the sulfonylureas) was available for the treatment of type 2 diabetes in the early 1990s, new classes of oral antidiabetic agents were developed. The thiazolidinedione class of medications was first introduced to the United States when troglitazone was marketed during early 1997. Rosiglitazone, approved by the FDA during the spring of 1999, was the second thiazolidinedione to be marketed in the United States. Similar to troglitazone, rosiglitazone improves insulin sensitivity in patients with Non-Insulin-Dependent Diabetes Mellitus by activating peroxisome proliferator-activated receptor-gamma (PPARγ) receptors in adipose tissues and skeletal muscles.

Non-Insulin-Dependent Diabetes Mellitus (NIDDM) is a form of diabetes where utilization of insulin is not the first line therapy. It occurs predominantly in adults, in whom production of insulin is available for use, yet a defect exists in insulin-mediated utilization and metabolism of glucose and peripheral tissues. For some people with diabetes, a mutation in the genes coding for insulin, for insulin receptor and/or for insulin-mediated signal transduction factors leads to ineffective insulin and/or insulin-mediated effects, impairing the utilization or metabolism of glucose.

The pathophysiology of non-insulin-dependent diabetes mellitus consists of three major components, (1) peripheral insulin resistance; (2) increased hepatic glucose production; and (3) impaired insulin secretion. Intense research has been devoted to each of these areas, independently, in order to determine which abnormality is primary and which are secondary.

When focussing on peripheral insulin resistance, the drug of choice is a thiazolidinedione, which is a type of insulin-sensitizing agent.

The thiazolidinedione chemical series has been shown to reverse insulin resistance in patients with NIDDM and impaired glucose tolerance, and can enhance insulin action in numerous genetic and acquired rodent models of insulin resistance. The antihyperglycemic effects of thiazolidinediones result from the ability to increase insulin dependent glucose disposal and reduce hepatic glucose production. It is believed that, by enhancing insulin action, thiazolidinedione treatment results in euglycemia at a lower circulating insulin level. In this regard, studies in normal and diabetic rodents and human clinical trials have not revealed hypoglycemia as a complication of thiazolidinedione therapy. On the other hand, administration of these drugs to normal or insulin-deficient diabetic animals failed to alter plasma glucose or insulin or glucose tolerance, although insulin sensitivity was nevertheless increased.

The effects of thiazolidinediones on glucose disposal are thought to result from insulin sensitization, indicating an absolute requirement for insulin. Thiazolidinedione treatments are based on the assumption that if peripheral insulin resistance is improved, increased hepatic glucose production and impaired insulin secretion will be alleviated in due course.

It has been observed that rosiglitazone markedly increased body weight gain (E Chaput et al, Biochem Biophys Res Commun 2000 May 10;271 (2):445-50). This side effect renders rosiglitazone monotherapy an undesirable prophylactic measure in the treatment of NIDDM.

As already explained, rosiglitazone is a PPARγ activator or agonist. In the present invention, the term agonist or activator is used equally to designate a compound that can activate a PPAR receptor.

PPARγ is a subtype of the PPAR (Peroxisome Proliferator Activated Receptor) family. PPARγ is predominantly expressed in white adipose tissue in rodents. Its expression is induced early during the course of differentiation of several preadipocyte cell lines. In fibroblasts, forced expression of PPARγ in the presence of an agonist such as a thiazolidinedione results in differentiation to an adipocyte phenotype.

Other activators of PPARs are effective drugs to improve the metabolic abnormalities linking hypertriglyceridemia to diabetes, hyperglycemia, insulin-resistance, and atherosclerosis.

Among them, fibrates can be cited as PPARα activators or agonists.

PPARα is another subtype of the PPAR family. PPARα is predominantly expressed in tissues catabolizing high amounts of fatty acids, such as liver, heart and brown adipose tissue. Activated PPARs form heterodimers with RXR (Retinoid X Receptor) and the heterodimer binds to a specific response element, termed PPRE (PPAR Response Element), in the regulatory regions of target genes and subsequently alters their transcription. The majority of the genes whose expression is under control of PPARα code for proteins involved in intra- and extracellular lipid metabolism, such as acyl coA oxidase, acyl-coA synthetase and apolipoproteins A-I, A-II and C-III.

Fibrates have been documented to lower plasma triglycerides and cholesterol levels and to be beneficial in the prevention of ischemic heart disease in individuals with dyslipidemia. They can also modestly decrease elevated fibrinogen and PAI-1 levels. Fibrate compounds, e.g., gemfibrozil, fenofibrate, bezafibrate, and ciprofibrate, elevate the level of plasma HDL cholesterol.

Methods and compositions presented as useful for the management of type 2 diabetes with PPAR modulators, are disclosed in WO98/05331.

The pharmacological profile of a PPARα activator, fenofibrate, and a PPARγ activator, rosiglitazone, was compared (E Chaput et al, Biochem Biophys Res Commun 2000 May 10;271(2):445-50) on serum parameters, target gene expression, and body weight gain in (fa/fa) fatty Zucker rats and db/db mice as well as their association in db/db mice. Fenofibrate faithfully modified the expression of PPARα responsive genes. Rosiglitazone increased adipose tissue aP2 mRNA in both models while increasing liver acyl CoA oxidase mRNA in db/db mice but not in fatty Zucker rats. Both drugs lowered serum triglycerides yet rosiglitazone markedly increased body weight gain while fenofibrate decreased body weight gain in fatty Zucker rats. KRP 297, which has been reported to be a PPARα and γ co-activator, also affected serum triglycerides and insulin in fatty Zucker rats although no change in body weight gain was noted. It was further observed that in db/db mice, rosiglitazone significantly increased body weight gain by 22% while the latter was non-significantly reduced by 10% by fenofibrate, and co-administration of fenofibrate and rosiglitazone did not reduce the weight gain induced by rosiglitazone.

It therefore is an objective of the present invention to provide a method for treating weight gain associated with a treatment by a PPARγ agonist such as rosiglitazone.

In accomplishing this and other objectives, there has been provided, in accordance with one aspect of the present invention, a therapeutic method comprised of co-administering a pharmacologically effective dose of a PPARα agonist and a PPARγ agonist, such that the weight gain associated with the PPARγ agonist treatment is decreased. In this respect, it was surprisingly found that co-administration of a PPARα agonist and a low dose of PPARγ agonist, was at least as effective as a higher dose of this PPARγ agonist alone in lowering blood glucose, and furthermore with less weight gain

By "PPARα agonist" is meant a compound or composition which when combined with PPARα directly or indirectly (preferably binding directly to PPARα) stimulates or increases an *in vivo* or *in vitro* reaction typical for the receptor, e.g. transcriptional regulation activity, as measured by an assay known to one skilled in the art, including, but not limited to, the "co-transfection" or "cistrans" assays described or disclosed in U.S. Patent Nos. 4,981,784, 5,071,773, 5,298,429, 5,506,102, W089/05355, WO91/06677, W092/05447, WO93/11235, WO93/23431, WO94/23068, WO95/18380, CA 2,034,220, and Lehmann, et al., J. Biol. Chem. 270:12953-12956 (1995), which are incorporated by reference herein. PPARα agonists may also be identified according to an assay described in US Patent 6,008,239.

A preferred PPARα agonist is a fibrate compound including, but not limited to, gemfibrozil, fenofibrate, bezafibrate, clofibrate, ciprofibrate, and analogues, derivatives and pharmaceutically acceptable salts thereof. PPARα compounds disclosed in Tontonez et al., Cell 79:1147-1156 (1994), Lehmann et al., J. Biol. Chem. 270(22):1-4, 1995, Amri et al., J. Lipid Res. 32:14491456 (1991), Kliewer et al., Proc. Natl. Acad. Sci. USA 94:4318-4323 (1997), Amri et al., J. Lipid Res. 32:1457-1463, (1991) and Grimaldi et al., Proc. Natl. Acad. Sci. USA 89:10930-10934 (1992) are incorporated by reference herein. PPARα agonist compounds described in US Patent 6,008,239, WO 97/27847, WO 97/27857, WO 97/28115, WO 97/28137 and WO 97/28149 are further incorporated by reference herein. Certain fibrate compounds as described in WO92/10468 and WO01/80852 are also incorporated by reference herein.

In the present invention, fibrates include fibric acid derivatives and pharmaceutically acceptable salts and esters of such fibric acid derivatives. Fibric acid derivatives lower the levels of triglyceride-rich lipoproteins, such as VLDL, raise HDL levels, and have variable effects on LDL levels. The effects on VLDL levels appear to result primarily from an increase in lipoprotein lipase activity, especially in muscle. This leads to enhanced hydrolysis of VLDL triglyceride content and an enhanced VLDL catabolism. Fibric acid agents also may alter the composition of the VLDL, for example, by decreasing hepatic production of apoC-III, an inhibitor of lipoprotein lipase activity. These compounds are also reported to decrease hepatic VLDL triglyceride synthesis, possibly by inhibiting fatty acid synthesis and by promoting fatty acid oxidation.

Fenofibrate is commercially available as TricorTM capsules. Each capsule contains 67 mg of micronized fenofibrate.

Clofibrate is commercially available as Atromid-S capsules. Each capsule contains 500 mg of clofibrate. Clofibrate lowers elevated serum lipids by reducing the very low-density lipoprotein fraction rich in triglycerides. Serum cholesterol may be decreased. It may inhibit the hepatic release of lipoproteins (particularly VLDL) and potentiate the action of lipoprotein lipase. The recommended daily dose of clofibrate is 2 g, administered in divided doses.

Gemfibrozil is commercially available as Lopid tablets. Each tablet contains 600 mg of gemfibrozil. Gemfibrozil is a lipid regulating agent that decreases serum triglycerides and very low density lipoprotein cholesterol, and increases high density lipoprotein cholesterol. The recommended daily dose of gemfibrozil is 1200 mg, administered in two divided doses.

According to the present invention, the preferred fibrate is fenofibrate.

By "PPARγ" agonist is meant a compound or composition which when combined with PPARγ directly or indirectly (preferably binding directly to PPARγ) stimulates or increases an *in vivo* or *in vitro* reaction typical for the receptor, e.g., transcriptional regulation activity, as measured by an assay known to one skilled in the art, including, but not limited to, the "co-transfection" or "cistrans" assays described or disclosed in U.S. Patent Nos. 4,981,784, 5,071,773, 5,298,429, 5,506,102, WO89/05355, WO91/06677, WO 92/05447, WO93/11235, WO93/23431, WO94/23068, WO95/18380, CA 2,034,220, and Lehmann, et al., J. Biol. Chem. 270:12953-12956 (1995), which are incorporated by reference herein.

A preferred PPARγ agonist is a thiazolinedione compound, including but not limited to, rosiglitazone, pioglitazone, ciglitazone, englitazone, darglitazone and analogues, derivatives and pharmaceutically acceptable salts thereof. PPARγ compounds disclosed in Tontonez et al., Cell 79:1147-1156 (1994), Lehmann et al., J. Biol. Chem. 270(22):1-4, 1995, Amri et al., J. Lipid Res. 32:14491456 (1991), Kliewer et al., Proc. Natl. Acad. Sci. USA 94:4318-4323 (1997), Amri et al., J. Lipid Res. 32:1457-1463, (1991) and Grimaldi et al., Proc. Natl. Acad. Sci. USA 89:10930-10934 (1992) are incorporated by reference herein.

According to the present invention, the preferred thiazolinedione compounds are rosiglitazone and pioglitazone, rosiglitazone being especially preferred.

A PPARα agonist can be used, in combination with a PPARγ agonist, to treat the weight gain associated with a PPARγ agonist treatment, optionally with other therapies, by improving lipidic control.

The invention includes a method of decreasing the body weight gain associated with a PPARγ agonist treatment, comprising co-administering an effective dosage of a PPARα agonist and a PPARγ agonist. The PPARα agonist used in this method may be a fibrate selected from the group consisting of gemfibrozil, fenofibrate, bezafibrate, clofibrate and ciprofibrate, and the PPARγ agonist used may be a thiazolinedione selected from the group consisting of rosiglitazone and pioglitazone.

In another embodiment, the invention includes a method of decreasing the weight gain associated with a PPARγ agonist treatment, comprising co-administering an effective dosage of a PPARα agonist and a PPARγ agonist, where the effective dosage of the PPARα agonist is in the range of about 10 to about 3000 mg per day, preferably in the range of about 50 to about 300 mg per day.

In another embodiment, the effective dosage of the PPARγ agonist is in the range of about 0.1 to about 100 mg per day, preferably in the range of about 0.5 to about 50 mg per day, more preferably of about 0.5 to about 10 mg per day, even more preferably of about 0.5 to about 3 mg per day, e.g. 0.5, 1.0, 1.5, 2.0, 2.5 and 3.0 mg per day.

In another embodiment, the PPARα agonist and the PPARγ agonist are administered simultaneously, in a method of decreasing the weight gain associated with the PPARγ agonist treatment, comprising co-administering an effective dosage of a PPARα agonist and a PPARγ agonist.

In another embodiment of a method of decreasing the weight gain associated with the PPARγ agonist treatment, the PPARα agonist and the PPARγ agonist are administered sequentially.

In another embodiment, the invention includes the use of a PPARα agonist, a PPARγ agonist and a pharmaceutically acceptable carrier for the manufacture of a medicament for decreasing the body weight gain associated with a PPARγ agonist treatment. In another embodiment, the PPARα agonist is a fibrate selected from the group consisting of gemfibrozil, fenofibrate, bezafibrate, clofibrate and ciprofibrate. In a further embodiment, the PPARγ agonist is a thiazolinedione selected from the group consisting of rosiglitazone and pioglitazone.

As demonstrated in the present specification, the use of a PPARα agonist and a PPARγ agonist, has led to favorably unexpected results. Studies were designed to evaluate the effects of a PPARα agonist and a PPARγ agonist as a combination therapy, on weight gain in diabetic rats. The data showed that the weight gain associated with the PPARγ agonist monotherapy was decreased when a PPARα agonist was administered in conjunction therewith. The data further showed that a low dose of PPARγ agonist was as effective as a high dose of this agonist.

As used in this application, "co-administration" means the administration of two or more compounds to the same patient, within a time period of up to about two to about twelve hours. For example, co-administration encompasses (1) simultaneous administration of a first and second compound; (2) administration of a first compound, followed by administration of a second compound about 2 hours after administration of the first compound; and (3) administration of a first compound, followed by administration of a second compound about 12 hours after administration of the first compound. As described herein, the present invention encompasses co-administration of a PPARα agonist and a PPARγ agonist to a patient.

Rosiglitazone in the present invention is defined as a compound of the class of thiazolidinediones: a class of compounds which work by enhancing insulin action and promoting glucose utilization in peripheral tissue. They apparently work by enhancing insulin action and thus promoting glucose utilization in peripheral tissues, possibly by stimulating non-oxidative glucose metabolism in muscle, and suppressing gluconeogenesis in the liver.

Rosiglitazone maleate is sold under the trademark *Avandia™* and is used in the management of type 2 diabetes mellitus (also known as non-insulin-dependent diabetes mellitus (NIDDM) or adult-onset diabetes).

Chemically, rosiglitazone maleate is (±)-5-[[4-[2-(methyl-2-pyridinylamino)ethoxy]phenyl]methyl]-2,4-thiazolidinedione, (Z)-2-butenedioate (1:1). The molecular formula is C₁₈H₁₉N₃O₃S C₄H₄O₄. The molecule has a single chiral center and is present as a racemate. Due to rapid interconversion, the enantiomers are functionally indistinguishable.

Rosiglitazone is described in US Patent 5,002,953, incorporated by reference herein.

Pioglitazone hydrochloride is sold under the trade name *Actos*^{*TM*} and is used in the management of type 2 diabetes mellitus.

Chemically, pioglitazone is (±)-5-[[4-[2-(5-ethyl-2-pyridinyl)ethoxy]phenyl]methyl]-2,4-thiazolidinedione. The molecule has one chiral center, but is produced as a racemate. The individual enantiomers have similar pharmacological properties.

According to the present invention, a preparation is defined as the formulation of the active compound with encapsulating material as a carrier providing a capsule in which the active component with or without other carriers, is surrounded by a carrier, which is thus in association with it. This includes tablets, powders, capsules, pills, cachets, and lozenges which can be used as solid dosage forms suitable for oral administration.

An effective dosage is defined in the present invention as the amount of a compound that prevents or ameliorates adverse conditions or symptoms of disease(s) or disorder(s) being treated. With respect to the PPARγ agonist and the PPARα agonist, effective dosage means a pharmacological dose in the range defined above. With respect to fibrates, the skilled artisan will understand and appreciate that the effective dosage of a given fibrate will vary with the potency of the fibrate.

The present invention relates to the unexpected discovery that co-administration of a PPARα agonist and a PPARγ agonist exerts beneficial effects on the weight gain induced by a PPARγ agonist treatment.

The invention includes a method of decreasing the body weight gain associated with a PPARγ agonist treatment, comprising co-administering an effective dosage of a PPARα agonist and a PPARγ agonist.

As will be shown in the example, the applicant unexpectedly found that a low dosage of a PPARγ agonist, which does not permit a normalization of the glycemia, when associated with a PPARα agonist, was at least as effective as a higher dose of this PPARγ agonist alone in lowering blood glucose, and that furthermore it reduces significantly the weight gain induced by the PPARγ agonist.

In this method, the effective dosage of both agonists is as defined above.

In the method of the invention, the PPARα agonist and the PPARγ agonist can be administered simultaneously, or sequentially. In a preferred embodiment of the invention, the PPARα agonist and the PPARγ agonist are administered simultaneously, more preferably in one formulation containing both compounds.

Pharmaceutical formulations of the PPARα agonist and/or the PPARγ agonist molecules can be prepared according to known methods. The preferred route of administering the PPARα agonist and the PPARγ agonist is mucosal administration, most preferably oral administration.

For preparing pharmaceutical compositions containing a PPARα agonist and/or a PPARγ agonist, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component. In tablets, the active component is mixed with the carrier having the necessary binding properties in suitable proportions and compacted in the shape and size desired. The powders and tablets preferably contain from five or ten to about seventy percent of the active compound. Suitable carriers are magnesium carbonate, magnesium stearate, talc, sugar, pectin, dextrin, starch, gelatin, tragacanth, methylcellulose, sodium carboxymethylcellulose, a low melting wax, cocoa butter, and the like.

Liquid form preparations include solutions, suspensions, and emulsions, for example, water or water propylene glycol solutions. For parenteral injection liquid preparations can be formulated in solution e.g. in aqueous polyethylene glycol solution.

Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

A further embodiment of the invention is related to the use of a PPARα agonist, or a PPARα agonist and a PPARγ agonist, and a pharmaceutically acceptable carrier for the manufacture of a medicament for decreasing the body weight gain associated with a PPARγ agonist treatment. Such a use is especially valuable in the treatment of type 2 diabetes mellitus.

The medicament can be the pharmaceutical preparation as defined above; the PPARα agonist is preferably a fibrate selected from the group consisting of gemfibrozil, fenofibrate, bezafibrate, clofibrate, ciprofibrate, fenofibrate being especially preferred; and the PPARγ agonist is preferably a thiazolinedione selected from the group consisting of rosiglitazone and pioglitazone, rosiglitazone being especially preferred.

The invention is further illustrated by the following example, which is not to be construed as limiting, but merely as an illustration of some preferred features of the invention.

### EXAMPLE: Effect of a PPARα agonist and a PPARγ agonist co-administration on body weight and on glycemia

This study was designed to evaluate the effects of using a combination of a PPARγ agonist, rosiglitazone, and a PPARα agonist, fenofibrate, for the treatment of diabetes and, in addition, if this combination therapy would prevent the body weight gain that is associated with the rosiglitazone treatment.

### METHOD

### Animals:

Male homozygous Zucker rats of 9 to 11 weeks of age and their lean controls were received from Iffa-Credo (France). They were put into individual cages in a temperature-, humidity- and light- controlled room (21-23°C, 12-12h light-dark cycle). They were fed with a standard laboratory diet and had free access to water. After acclimatization, they were randomized into groups of 10, based on body weight.

The experimental groups were:
Group 1= lean rats, untreated;
Group 2 = obese rats, treated with the vehicle p.o., twice daily (at 8 a.m. and 8 p.m.);
Group 3 = obese rats, treated with Fenofibrate, 100 mg/kg, p.o., twice daily (at 8 a.m. and 8 p.m.);
Group 4 = obese rats, treated with Rosiglitazone, 0.3 mg/kg, p.o., twice daily (at 8 a.m. and 8 p.m.);
Group 5 = obese rats, treated with Rosiglitazone, 3.0 mg/kg, p.o., twice daily (at 8 a.m. and 8 p.m.);
Group 6 = obese rats, treated with Fenofibrate, 100 mg/kg and Rosiglitazone, 0.3 mg/kg, p.o., twice daily (at 8 a.m. and 8 p.m.);
Group 7 = obese rats, treated with Fenofibrate, 100 mg/kg and Rosiglitazone, 3.0 mg/kg, p.o., twice daily (at 8 a.m. and 8 p.m.).

The following parameters were monitored: glycemia (mg/dl) and body weight gain (g). The results are summarized in Tables 1 and 2.

**Table 1:**

| **results after 41 days of treatment** | | |
|---|---|---|
| **Group** | **Glycemia** | **Body Weight** |
| 1 | 131.3±4.8 | 328.4±4.5 |
| 2 | 624.4±32.1 | 386.2±5.9 |
| 3 | 222.2±53.1 | 357.0±7.9 |
| 4 | 274.6 ± 56.4 | 538.5±15.8 |
| 5 | 119.6±5.9 | 601.4±9.3 |
| 6 | 159.1 ± 34.5 | 400.8 ± 4.3 |
| 7 | 133.3 ± 5.7 | 466.5 ± 4.6 |

**Table 2:**

| **results after 69 days of treatment** | | |
|---|---|---|
| **Group** | **Glycemia** | **Body Weight** |
| 1 | 153.5±4.6 | 371.3±4.9 |
| 2 | 733.3 ± 22.8 | 397.1±3.7 |
| 3 | 372.5 ± 78.5 | 380.6 ± 13.7 |
| 4 | 446.0±52.4 | 615.2±25.9 |
| 5 | 151.4±6.5 | 721.0± 12.3 |
| 6 | 262.5 ± 56.9 | 435.2±10.1 |
| 7 | 181.5±10.4 | 548.6±8.6 |

These data demonstrate the following:
- As far as body weight gain is concerned, rosiglitazone alone produced a dose-dependent increase in body weight gain. A significant reduction in body weight gain was seen upon co-administration of fenofibrate with rosiglitazone, body weight gain control being better at the low rosiglitazone dose of 0.3 mg/kg.
- As far as glycemia is concerned, (1) euglycemia was attained with 3.0 mg/kg rosiglitazone alone while the glycemia was not controlled with the 0.3 mg/kg dose between day 41 and day 69, and (2) euglycemia was perfectly maintained at day 69 with the co-administration of fenofibrate and rosiglitazone (3.0 mg/kg), and still achieved in 8 rats out of 10 when 0.3 mg/kg rosiglitazone was co-administered with fenofibrate (mean values of these 8 rats : 170.7 ± 6.3 mg/dl).

Therefore, the co-administration of a PPARα agonist such as fenofibrate and a PPARγ agonist such as rosiglitazone makes it possible not only to control glycemia but also to reduce the body weight gain associated with the PPARγ agonist treatment. A low effective dosage of the PPARγ agonist is especially suitable to achieve both objectives.

## Claims

**1.** A method of decreasing the body weight gain associated with a PPARγ agonist treatment, comprising co-administering an effective dosage of a PPARα agonist and a PPARγ agonist.

**2.** The method according to claim 1, wherein the PPARα agonist is a fibrate selected from the group consisting of gemfibrozil, fenofibrate, bezafibrate, clofibrate and ciprofibrate.

**3.** The method according to claim 2, wherein the fibrate is fenofibrate.

**4.** The method according to one of claims 1 to 3, wherein the effective dosage of the PPARα agonist is in the range of about 10 to about 3000 mg per day.

**5.** The method according to one of claims 1 to 4, wherein the PPARγ agonist is a thiazolinedione selected from the group consisting of rosiglitazone and pioglitazone.

**6.** The method according to claim 5, wherein the thiazolinedione is rosiglitazone.

**7.** The method according to one of claims 1 to 6, wherein the effective dosage of the PPARγ agonist is in the range of about 0.1 to about 100 mg per day.

**8.** The method according to one of claims 1 to 7, wherein the PPARα agonist and the PPARγ agonist are administered simultaneously.

**9.** The method according to one of claims 1 to 7, wherein the PPARα agonist and the PPARγ agonist are administered sequentially.

**10.** Use of a PPARα agonist and a pharmaceutically acceptable carrier for the manufacture of a medicament for decreasing the body weight gain associated with a PPARγ agonist treatment.

**11.** Use of a PPARα agonist, a PPARγ agonist and a pharmaceutically acceptable carrier for the manufacture of a medicament for decreasing the body weight gain associated with a PPARγ agonist treatment.

**12.** The use according to claim 10 or 11, wherein the PPARα agonist is a fibrate selected from the group consisting of gemfibrozil, fenofibrate, bezafibrate, clofibrate, ciprofibrate.

**13.** The use according claim 12, wherein the fibrate is fenofibrate.

**14.** The use according to one of claims 10 to 13, wherein the PPARγ agonist is a thiazolinedione selected from the group consisting of rosiglitazone and pioglitazone.

**15.** The method according to claim 14, wherein the thiazolinedione is rosiglitazone.

**14.** The use according to one of claims 10 to 15, wherein the PPARα agonist and the PPARγ agonist are administered simultaneously or sequentially.

**15.** A pharmaceutical composition comprising a PPARα agonist, a PPARγ agonist and a pharmaceutically acceptable carrier, wherein the effective dosage of the PPARγ agonist is in the range of about 0.5 to about 3 mg per day.

**16.** The pharmaceutical composition according to claim 15, wherein the PPARα agonist is a fibrate selected from the group consisting of gemfibrozil, fenofibrate, bezafibrate, clofibrate, ciprofibrate.

**17.** The pharmaceutical composition according claim 16, wherein the fibrate is fenofibrate.

**18.** The pharmaceutical composition according to one of claims 15 to 17, wherein the effective dosage of the PPARα agonist is in the range of about 10 to about 3000 mg per day

**19.** The pharmaceutical composition according to one of claims 15 to 18, wherein the PPARγ agonist is a thiazolinedione selected from the group consisting of rosiglitazone and pioglitazone.

**20.** The pharmaceutical composition according to claim 19, wherein the thiazolinedione is rosiglitazone.
